(19) Europäisches Patentamt / European Patent Office / Office européen des brevets

(11) **EP 4 171 731 B1**

(12) **EUROPEAN PATENT SPECIFICATION**

(45) Date of publication and mention
of the grant of the patent:
**11.03.2026 Bulletin 2026/11**

(21) Application number: **21731406.1**

(22) Date of filing: **27.05.2021**

(51) International Patent Classification (IPC):
***A61N 2/02*** *(2006.01)*

(52) Cooperative Patent Classification (CPC):
**A61N 2/02**

(86) International application number:
**PCT/EP2021/064169**

(87) International publication number:
**WO 2022/002493 (06.01.2022 Gazette 2022/01)**

(54) **DEVICE FOR MAGNETIC FIELD THERAPY**

VORRICHTUNG ZUR MAGNETFELDTHERAPIE

DISPOSITIF POUR LA THÉRAPIE PAR CHAMP MAGNÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2020 DE 102020117033**

(43) Date of publication of application:
**03.05.2023 Bulletin 2023/18**

(73) Proprietor: **Gleim, Niki Reiner
9485 Nendeln (LI)**

(72) Inventor: **KAFKA, Wolf A.
82288 Kottgeisering (DE)**

(74) Representative: **Epping - Hermann - Fischer
Patentanwaltsgesellschaft mbH
Schloßschmidstraße 5
80639 München (DE)**

(56) References cited:
**EP-A1- 0 594 655          EP-A1- 2 050 481
DE-U1- 202016 008 331**

**Description**

Technical field of the invention

The invention relates to a device for magnetic field therapy as described, for example, in EP 2050481 B1.

Prior art

Since the beginning of the 1970s, devices have been known, some of which are also used routinely primarily in the field of orthopedics in clinics, medical practices and in home use for therapeutic purposes.

A generator is used to control a magnetic field generating device, whereby the generator controls the magnetic field generating device in such a way that the magnetic field consists of a large number of basic pulses or main pulses which are characteristically shaped in terms of time interval and amplitude profile and whose pulse frequency is usually between 0 and 1000 Hz. Such a main pulse can be e.g. by sinusoidal, trapezoidal, sawtooth shaped (EP 0594655 B1, EP 0729318 B1 or as in EP 0995463 B1) by an on average exponentially increasing sinusoidal modulated field intensity curve with magnetic flux densities in the range of nanoTesla to several milliTesla, in such a way that the main pulses, as for example in EP 0995463 B1, are composed of a series of temporally successive sub-pulses, which differ in their amplitudes and/or rise or fall slopes, and thus ultimately also in their individual duration.

The generation of the magnetic fields is often carried out by one or more electric coils which are also controlled independently of each other. From the multitude of devices for electromagnetic therapy, documents correspondingly oriented to the generation of time-varying field intensities can be found, for example, in EP 0995463 B1.

Today, therapeutic application is usually performed non-invasively for reasons of surgical effort and the associated risks.

The influence on the biological system is based, according to common understanding, on an interaction of the magnetic and electric field components generated by the devices, the energy components of which are still unknown. The triggered physiological and biological effects are based on the direct, by magnetic or/and according to the principle of induction (Maxwell's equations) of the indirect, by electrical force effects caused influence (energetic activation) of the physical-chemical reactivity of the ionic, atomic and molecular building blocks involved in the natural and self-preserving regulatory mechanisms, in the following referred to as molecular structures.

For example, it is described that the use of the special device EP 0995463 B1 in relation to untreated biological objects as controls leads to a number of differentiated physical-physiological processes, such as the significant activation of processes

- in the formation of energy-rich compounds, in particular adenosine triphosphate (ATP) and bis-2-3-phospoglycerate (BPG) in human erythrocytes;
- to improve the functional state of the microcirculation, in particular with regard to blood flow (especially also in diabetes-related circulatory disorders) and oxygen utilization, as well as
- in the course of protective mechanisms, especially with regard to an accelerated course of infectiously triggered leukocyte immune defense reactions supported by complex interplay of signaling and adhesion molecules;
- in protection against chemical stress factors, especially the reduction of chemically (by the teratogen cyclophosphamide) induced malformations in the ontogenesis of warm-blooded vertebrate embryos;
- in reparative processes, in particular with regard to improved healing of wounds produced by default;
- in anti-oxidative regulations, especially with regard to enzymatically and spectrophotometrically determined accelerated reduction turnover rates;
- in performance enhancement in top-level sports;
- of replication and proliferation mechanisms, in particular with regard to a significant reduction of tumor growth in thymus-free but not in comparatively examined normal mice;
- protein formation and activation, especially with regard to differential up- and down-regulation of gene-expressed protein levels;
- psychovegetative processes, in particular the reduction of (dental) anxiety by local electromagnetic stimulation of the solar plexus immediately preceding dental treatment;
- the reduction of lumbar-initiated subsequent reactions, especially the reduction of movement pain, insomnia and anxiety;
- the analgesic effect, especially with regard to the reduction of polyneuropathic pain states as a consequence of oxidative stress after chemotherapy.

Underlying problem

All devices known so far for the treatment of the human body lead to the desired accelerating effect of healing processes, but their effect can still be improved. Without taking into account whether a patient actually benefits from such a treatment, statements about it in the form of so-called surrogate parameter studies often refer only to the treatment of laboratory values such as parameters of the circulation, fat transport (LDL, HDL), lung function, bone density or other metabolic rates. This is problematic in that in order to achieve a significantly accelerated healing success, the application must be repeated frequently, often varying the field intensity ratios, which leads not only to an increased burden on the patient, but also to significantly higher treatment costs.

The relationship between the temporal intensity ratios of the applied electromagnetic fields and the respective induced biological effect is still insufficiently exploited in the device of EP 0995463 B1. According to the mentioned ideas about the effect, in particular the sub-pulses of the main pulse, which rise steeper and higher in their absolute intensity (independent of the sign) compared to the following sub-pulses, have in principle a lower potential for individual influencing of the activation energies modulating the biological reactions due to their amplitude and edge steepnesses. Simply put, the sub-pulses of a main pulse generated in immediate succession in EP 0995463 B1 are always smaller in amplitude and slope than the ones following them. Accordingly, and additionally due to the different electric field components according to Maxwell's equations, these provide only a correspondingly limited lower energetic contribution compared to the respective following sub-pulses in the sense of the desired activation of regulatory mechanisms spread as widely as possible.

With reference to the envelope curves described in EP 0995463 B1 as a connection of the upper and lower extreme values of the sub-amplitudes, it is added that the envelope curve closer to the coordinate axis increasingly moves away from it within the pulse train: Measured in terms of amplitude, successive sub-pulses would therefore have a lower activation potential compared to those in which the envelope curve described moves less "away" from the coordinate axis. - Accordingly, in the sense of the desired broad activation, the forms of the main and sub-pulses described in EP 0995463 B1 are still insufficiently graded in terms of duration and amplitude and amplitude sequence.

In a further development described in EP 2050481 B1, the intensity progression over time was adjusted so that the pulses are more finely adapted to the requirements of the therapy. The optimal form and sequence of the sub-pulses varies greatly from individual to individual. It depends on the type of tissue impacted by the field, the desired healing outcome, and the individual. The high proportion of rising and falling flank sections, caused by the large number of sub-impulses, is crucial in stimulating the exchange processes in the body's tissues.

Objective and solution

An object to be achieved is to specify an improved concept for magnetic field therapy with a driving signal characterized by a broader spectral composition.

This object is achieved by the independent claim, wherein advantageous further embodiments are specified in the dependent claims.

The improved concept ensures, for example, a broader physiological range of effects by addressing a broad band of electromagnetically activatable molecular structures.

The improved concept is thus oriented to the broadest possible energetic support of the complexly interconnected molecular regulatory processes that determine the normal course of life. Deviating from the usual symptom-oriented treatment methods, it accordingly pursues a holistic and thus preventive therapy concept, as well as a therapy concept oriented towards regeneration, maintenance and well-being.

The improved concept is based on the idea of continuously varying the sequence of different electro-magnetic stimulation signals during treatment, in particular by superposition of at least two similar stimulation signals, especially sequences of pulses with different parameters regarding pulse length and pulse repetition rate. This is done in particular by varying pulse length and pulse repetition rate in at least one of the sequences to be superimposed. The variation of the sequence of such signal sequences generated by superposition, interrupted by possible pauses, can also be used. Advantageously, compared to conventional methods, different tissue parts can be exposed to temporally varying signal patterns in the course of a treatment in this way (due to intensity or range). Depending on the type of superposition and signal sequence, in particular also periodically or rhythmically repeating physiological and bio-rhythmic processes such as respiratory and heart rates, sleep rhythms, etc. could thus be influenced or supported.

According to the improved concept, for example, a device for influencing biological processes in a living tissue, in particular a human body, for applying a pulsating magnetic field to at least a part of the tissue comprises a field generating device for generating the pulsating magnetic field and a pulse generator for driving the field generating device. For this purpose, the pulse generator is adapted to output a signal sequence which is formed from a superposition of a first sequence of main pulses and at least one second sequence of main pulses whose pulse repetition rate is in each case between 0.1 and 1000 Hz, for example between 1 and 3 Hz as the lower limit and 200 to 300 Hz as the upper limit.

Thereby, each main pulse comprises a pulse length. The pulse length and the pulse repetition rate of the main pulses of the second sequence of main pulses are different from the pulse length and the pulse repetition rate of the main pulses of the first sequence of main pulses. Further, the pulse length of the main pulses of the second sequence of main pulses changes monotonically in a time-dependent course. For example, the pulse length decreases monotonically or increases monotonically. The pulse repetition rate of the main pulses of the second sequence of main pulses also changes monotonically over time, in the opposite direction to the change in pulse length. For example, the pulse repetition rate monotonically increases or monotonically decreases. Thus, if the pulse length increases, the pulse repetition rate decreases and vice versa.

Therein an amplitude waveform of a main pulse comprises the following function y(x):

$$y(x) = k1 + k2 \cdot e^{\sin(x^{k3}) + \sin((x \cdot k4)^{k5}) + x \cdot k6}.$$

Therein mean:

x = computational substitute for time t during a main pulse;
k1 = offset value;
k2 = amplitude factor, k2≠0;
k3 = exponent of x, k3≠0;
k4 = multiplication factor of x, k4≠0;
k5 = exponent of (x*k4), k5≠0;
k6 = multiplication factor of x;
wherein k1 - k6 are parameters which are freely selectable within certain limits to give different shapes to the amplitude waveform, wherein each main pulse is modulated with sub-pulses (11) by respective selection of the parameters.

The function describes an exponential modulation course essentially defined by the parameters k1, k2, k3, k4, k5 and k6, wherein this modulation course is governed by two SIN functions determined by the parameters k3, k4 and k5. Since a SIN function can only assume values between +1 and -1, the sum of the two SIN functions determining the e-function lies between -2 and +2. The e-function can therefore only assume values between $e^{-2} = 0.135$ and $e^2 = 7.39$, if one disregards the further term +x*k6, which then defines a further increase. The SIN functions themselves with the associated parameters are chosen in such a way that a fine gradation of the successive amplitude values in the sub-pulses defined with them is achieved with a high range of variation.

The selectable parameters k1 to k6 have the following meaning and effect on the signal sequence, in particular on the main pulses forming the signal sequence:

- k1 is a presettable basic amplitude value with which a basic signal value or bias value can be defined on which the main pulses "touch down" (zero line symmetry or zero line asymmetry). This basic value does not have to correspond to a fixed amplitude value, but can vary individually in the time sequence for each main pulse.
- k2 (k2≠0) is a multiplication factor for the e-function; the larger k2 is selected, the larger is the maximum achievable modulation amplitude value of a main pulse, which is added to the basic amplitude value. k1 and k2 can be limited - depending on k6 - in such a way that the permissible field strengths, which differ in individual countries, are not exceeded.
- k3 determines as a kind of time-dependent scaling factor together with k4 and k5 the time-dependent course (slope and amplitude) of the modulated signal or magnetization value of the individual sub-pulses. The larger the parameter k3 is chosen, the larger the "ripple" of the envelopes connecting the extreme values of the sub-pulses.
- Together with k3 and k5, k4 also determines the number and slope of the individual sub-pulses, but in particular, as a kind of density factor, the time sequence of the individual sub-pulses , (k4≠0); the larger the parameter k4 is selected, the more densely the individual sub-pulses are embedded in the individual "waves" of the envelopes of each main pulse.
- k5 is, similar to k3, a kind of temporal scaling and dimensioning factor, by which In conjunction with the parameters k3 and k4 the temporal modulation course (slope, amplitude, and density in the sequence within the "waves" described above) of the signal or magnetization value of the individual sub-pulses within a main pulse can be adjusted.
- k6 is a normalization and dimensioning factor for setting an average amplitude value which depends on k1 and k2 and can be fixed for the sequence of the individual main pulses. It specifies with which slope the amplitude of the main pulse increases or decreases during the "active" pulse duration. k6 thus also describes the average amplitude increase of the sub-pulses embedded in the main pulse. For example, values k6 > 0 lead to increasing amplitude values of the sub-pulses on average, and values k6 < 0 lead to decreasing amplitude values on average - especially illustrated by the envelopes connecting the extreme values of the sub-pulses. This has a particular effect on the envelope facing away from the abscissa axis. With k6 = 0, the amplitude values of the sub-pulses remain constant on average and the envelopes run parallel to the x-axis. The parameter k6 can be given as k6≠0.

The parameters of the function y(x) can be chosen, for example, with the properties k3 to k5 being integer and k1, k2 and k6 selected in decimal increments from the following ranges of values:

- 6 < k1, k2, k6 < 6 (particularly in steps of 0.1);
- 6 < k3, k5 < 6 (integer);
- 10< k4 < 10.

Accordingly, both the first sequence and the at least one second sequence each consist of a certain number of main pulses, each following said function y(x). However, the parameters k1 to k6 of the first sequence may differ from corresponding parameters of the second sequence, as well as between different main pulses within a sequence. The different pulse length results in particular from the used approach, how the arithmetic substitute x for the time t results.

A respective main pulse is composed, for example, of an amplitude sequence of sub-pulses with different edge steepnesses which remains constant on average or increases or decreases on average in the manner of a power function. Characterized by connecting lines of the extrema, also envelopes, of the individual sub-pulses, the main pulses can assume a pulse-shaped course depending on the selected conditions.

For example, the computational substitute x depends linearly on the time t.

For example, the computational substitute x for time t is defined as

$$x = x1 + \frac{x2-x1}{T_i} \cdot (t - t0_i),$$

with

x1 = initial value for x;
x2 = final value for x;
Ti = pulse length of a main pulse; and
t0i = start time of the main pulse.

Thus, a linear progression for the value x between the initial value x1 and the final value x2 ultimately results, independent of the pulse length of the main pulse concerned. In other words, the course of x between x1 and x2 is stretched or compressed by a changing pulse length.

In this case, the pulse length Ti of the main pulses of the second sequence of main pulses can change in a time-dependent course, for example, linearly, logarithmically or exponentially.

The pulse generator is used to drive the field generation device, wherein the pulse generator drives the field generation device via suitable current or voltage sequences in such a way that the pulsating magnetic field resulting from the signal sequence is composed in such a way that the pulsating magnetic field resulting from the signal sequence has as large a spectral width as possible.

In the case of voltage control, it must be ensured that the inductance of the coil means that the applied voltage signal does not result in a magnetic field signal that corresponds to the mathematical curve of the voltage signal, because the inductance of the coil reproduces the signal curve at higher frequencies with lower amplitudes. This can be counteracted, for example, by transferring the desired time function of the magnetic field into the frequency domain via a Fourier transformation, where the high frequencies are raised accordingly in order to then obtain the appropriate time signal for the voltage waveform.

Another possibility is to use a voltage-controlled current amplifier, which applies current signals to the coil with a waveform that corresponds to the desired magnetic field signal. The superposition of the first and second sequences to form the signal sequence also results in different spectral characteristics of the individual sequences due to the different pulse lengths and pulse repetition rates, which ultimately leads to a superposition of spectral characteristics in the signal sequence output by the pulse generator. Furthermore, the monotonic change in pulse length also results in a changing spectral property over time, so that interactions of different frequency components can be achieved in the pulsating magnetic field and thus in the impacted tissue.

Because of the changing pulse lengths and the resulting frequency shifts within the main pulses, changing frequency deviations between the signal of the first sequence and the signal of the second sequence thus follow. This, in turn, can lead to beat-like conditions, which result in further stimulation of the impacted tissue.

Due to the differential effects on tumor growth and gene expression, the effects cannot be explained with an improved microcirculation, but confirm and imply the assumption stated in the introduction that the electromagnetically induced biological effects are based on the activation of causally different molecular mechanisms. It is assumed that the different processes consequently require different amounts of energy for their activation. The distribution of the amplitudes, the embodiment of the slopes and the superposition of the sub-pulses are therefore of decisive importance, since with these parameters the intensity distribution over time is characterized. As a kind of electromagnetic agent, the temporal field intensity distributions are therefore of similar importance to the structure-activity relationship of active pharmaceutical ingredients.

Instead of chemical formulas, it could be quantified according to well-known rules of school mathematics as an additive superposition of sine and cosine components suitably superimposed with respect to frequency and amplitude, e.g. as a characteristic amplitude-frequency (Fourier) spectrum. The broader the amplitude-frequency spectrum of the applied fields, the broader the activation possibilities and thus ultimately the more efficient and broadly spread the expected biological effect would be.

However, even if a device with a broadband field application already contains many of the intensity time courses used in other devices, it should not be assumed that this also covers their effects. In addition - and thus similar to medication with multifunctional agents - synergistic effects (resulting from their interaction) must also be taken into account here. In this respect, the sequence of different electro-magnetic signal sequences of the type described - also interrupted by pauses - can also play a role.

In various embodiments, the main pulses of the first sequence of main pulses follow each other directly in the time-dependent course, in particular without a pause. Similarly, the main pulses of the second sequence of main pulses also follow one another immediately in the time-dependent course, in particular without a pause. This can result, for example, in the start times of the individual main pulses in the first and second sequences moving further apart with each main pulse, and thus also in the spectral components within the main pulses overlapping at different times in order to achieve the broadest possible excitation spectrum.

In alternative embodiments, the main pulses of the first and/or second sequence may also follow each other with pauses. These may comprise a constant length, or they may comprise a variable length corresponding, for example, to the change in pulse length.

In various embodiments, the pulse length of the main pulses of the first sequence of main pulses may remain constant, while the pulse length of the main pulses of the second sequence changes.

However, in various embodiments, it is also possible for the pulse length of the main pulses of the first sequence of main pulses to change monotonically over time, for example to increase monotonically. In this case, the pulse repetition rate of the main pulses of the first sequence of main pulses changes in the time-dependent course in the opposite direction to the change in the pulse length of these main pulses, for example increases monotonically or decreases monotonically. The variation in pulse length and pulse repetition rate in the first sequence of main pulses results in a further change in spectral characteristics over time, so that in turn a further broadening of the frequency spectrum acting on the tissue can be expected.

In various embodiments, the pulse generator is adapted to output the signal sequence as a first signal sequence and at least one further signal sequence in temporal succession, the at least one further signal sequence being different from the first signal sequence. The first and the at least one further signal sequence are thereby composed according to the principle described above, namely by superimposing two sequences of main pulses. For example, the signal sequences differ with respect to the pulse length of the main pulses of the first sequence and/or the pulse length of the main pulses of the second sequence and/or one or more of the parameters k1 to k6.

For example, the pulse generator is adapted to output the at least one further signal sequence after a defined pause time after the output of the first signal sequence.

The present device leads to a faster and broader excitation of the molecular interactions and metabolic processes involved in the regulatory mechanisms. With regard to the fundamental significance of these regulatory mechanisms, which are crucial for the course of life, such field excitation can thus achieve more beneficial effects in a wide variety of medical applications. For example, in relation to the processes for improving the functional state of the microcirculation and the associated increase in $O_2$ utilization, this leads to a further increase in the production of the energy carrier ATP, which energetically supports the processes of transcription, translation, formation and modulation of the activity state of proteins, and - as a further consequence - also to an accelerated setting of proteomics, i.e. a more efficient provision of the proteins modulating these regulations.

In particular, this can also lead to further improved support of the extremely complex sequence of immune defense reactions characterized by signaling molecules and various forms of adhesion molecules.

In conjunction with increased synthesis performance, increased $O_2$ utilization leads, inter alia, on the one hand to increased connective tissue and cartilage formation and additional vascularization and thus also enhances the formation of chondrocytes, which is strongly dependent on $O_2$ diffusion in the cartilage. Due to a shape-retaining and regeneration-promoting effect of this bone formation promoted by electromagnetic pulses, the organism is thus able to repair the necessary structures and injury- and disease-related disturbances in bone formation in an accelerated manner with a minimum of material and energy.

On the other hand, the bioelectrical effect of the induced tensions, in connection with an activation of the proton pumps supporting the ATP formation and further favored by the $O_2$ utilization, can lead to a mineralization of the connective tissue via an increased ion exchange. Because of the build-up and degradation processes of cartilage, which are closely coupled to bone metabolism, the device may also influence the calcium influx and efflux kinetics of chondrocytes, which are co-decisive for the consolidation of bone fragments.

The membranes of the membrane systems, which are important for intracellular and intercellular signaling and substance transfer, are affected either directly or by the potentials formed in the collagen, or also via a change in the microenvironment of the cell. This mechanism, possibly also as above in conjunction with electromagnetic support of proton transport mechanisms, is presumably based on electrochemical transmission that modifies cell activity by shifting the ionic atmosphere in the extracellular and thus also in the intracellular space. The capacitive charging of the cell membrane by the electrical component of pulsating electromagnetic fields represents a crucial factor in this process. Caused by the structural and charge shift in the membrane, especially in the area of the pores, there is the possibility of a permeability change with a resulting influence on passive ion transport and diffusion processes.

Due to the close coupling between surface reaction and transmembrane transport, other membrane-bound proteins, such as the Na-K pump, appear to be important receiver structures for the induced energy in addition to the proton pump. In this context, increased Na-K adenosine triphosphatase activity can cause increased sodium supply by the responsible ion pump. In this context, only excitation with an optimal amplitude profile of the main pulses according to the invention leads to excitation of the active transport complexes via presumably an increase in the surface concentration of the corresponding ions.

In its positive effect on the complexly interconnected, multilayered regulatory processes in the course of life, the list of advantageously supported individual mechanisms could be further extended in this way. Apart from the aforementioned general positive effect, also on competitive sports, the device can thus contribute quite generally to the optimized support of general natural protective, healing and maintenance processes and well-being.

In conjunction with the reduction of the occurrence of general chronic disorders, this invention could ultimately also further limit the development of general illness costs via its influencing of vital processes of substance and energy turnover and thus also the reduction of medications linked thereto.

What is particularly advantageous about the proposed device is that it can also lead to a stimulation of metabolic processes throughout the patient's body in the case of local treatment.

Good results can also be achieved if, during a treatment period, the parameters of the amplitude function y are not kept constant, but are varied according to a pattern tailored to the patient. For this purpose, groups of parameters are defined which are then applied successively in time periods to be selected.

The improved concept is explained in more detail below by means of exemplary embodiments with reference to the drawings. Here, elements of the same kind or elements of the same functions are designated with the same reference signs. Therefore, a repeated explanation of individual elements may be omitted.

In the drawings:

Figure 1    shows a schematic representation of an embodiment of a device for influencing biological processes,

Figure 2    shows a principled time-dependent course of a main pulse,

Figure 3    shows a principled time-dependent course of main pulses from a first and a second sequence before superposition,

Figure 4    shows a principled time-dependent course of a first and a second sequence before the superposition as well as of a signal sequence after the superposition, and

Figure 5    shows a principled time-dependent course of a sequence of superimposed signal sequences.

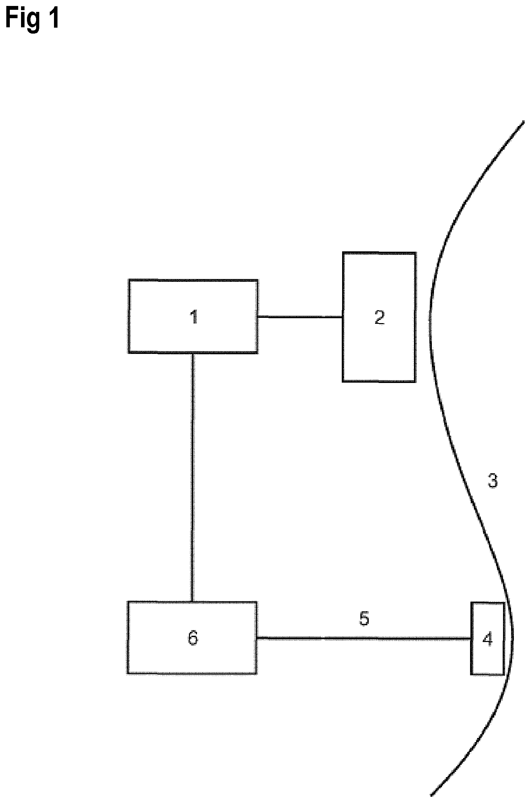

In detail, Figure 1 shows a device according to the improved concept, comprising at least one pulse generator 1, which generates a pulsating magnetic field in a coil 2, which takes effect in the living tissue 3, in particular in the body of a patient to be treated. In order to adjust, in particular optimize, the pulse parameters of the pulsating magnetic field in the pulse generator 1, an optional sensor 4 can detect certain body parameters. Such body parameters include, for example, temperature, blood pressure, pulse rate, skin resistance or blood oxygen content. The sensed parameter is fed via a feedback line 5 to a control unit 6, which evaluates the parameter and controls the pulse generator 1 accordingly. For improved optimization, it is possible to record and evaluate several body parameters simultaneously for optimization of the pulsating magnetic field. Depending on these effects, the control unit 6 can also automatically determine the optimal values for the parameters k1 to k6 in each case.

In addition, a sensor for detecting the frequency dependence of the effect of the field generating device 2 transmitted to the body can optionally be provided. From the differences, in particular in the spectral composition between the field energy generated by the field generation device and the spectrum detected by the sensor, the control unit determines the portion transmitted to the treated body. Depending on this effect, the control unit 6 itself determines the optimum values for the parameters k1 to k6. In such field generating devices 2, the field strengths can additionally be varied within the geometry of the field generating device 2.

With the device according to the improved concept, a pulsating magnetic field is generated in such a way that a signal sequence is delivered from the pulse generator 1 to the field generating device, which is formed from a superposition of a first sequence of main pulses and a second sequence of main pulses.

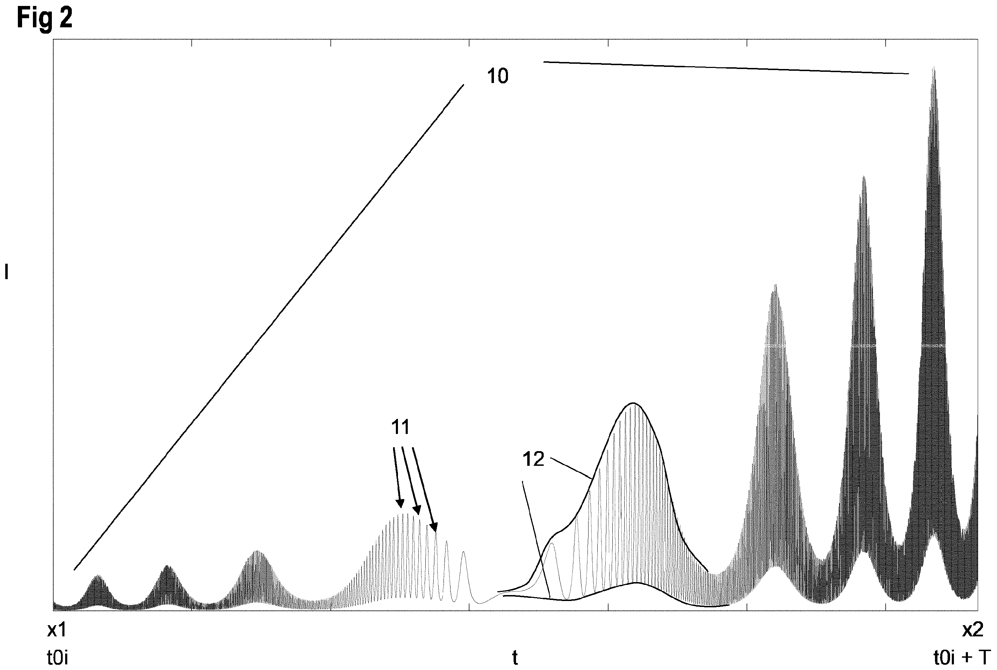

Figure 2 shows a principal time-dependent course of the intensity I of such a main pulse 10, which is modulated with sub-pulses 11. The main pulse 10 follows the already described function y(x)

$$y(x) = k1 + k2 \cdot e^{\sin(x^{k3}) + \sin((x \cdot k4)^{k5}) + x \cdot k6}.$$

With the parameters k1 to k6 already described. The main pulse 10 starts at time t0i and ends at time t0i+T, where T is a value for the pulse length. Since the function y(x) is based on the computational substitute x for the time t, this results in a course of the function between x1 and x2. The choice of the values of x1 and x2 is in principle arbitrary, but lies for example in the range from -10 to 0 for x1 and 0 to 10 for x2, thus for example x1 = -5 and x2 = +5. The curve of the intensity I of the main pulse 10 in Figure 2 shows that the density and thus also the steepness of the sub-pulses 11 following one another in the main pulse 10 and thus also the "ripple" (pulsations of the envelope curve 12 connecting their extremes) increases constantly depending on the respective x-values or t-values.

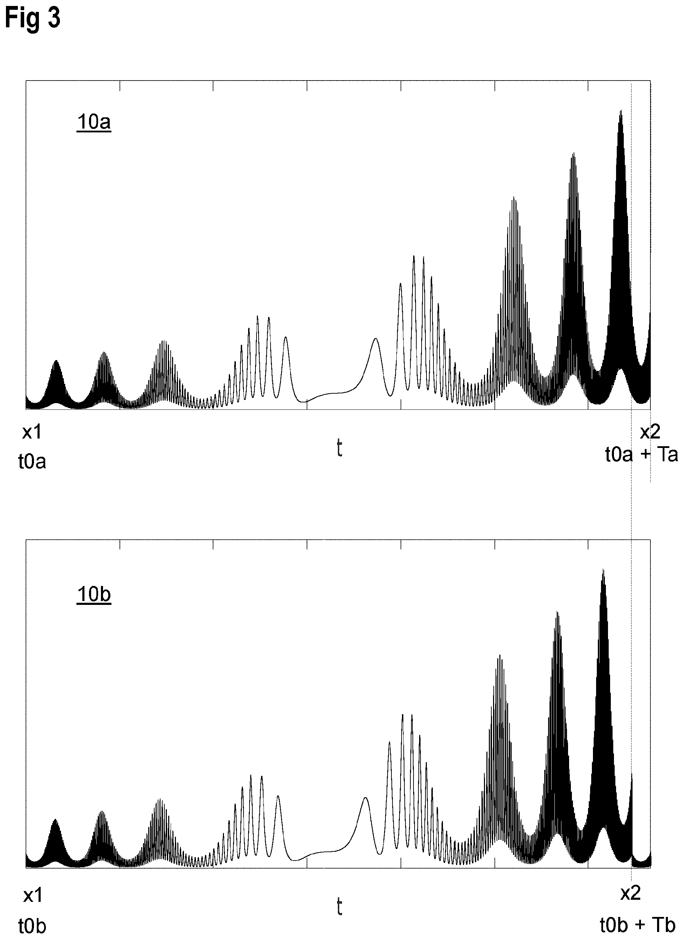

Figure 3 shows a principal time-dependent course of two main pulses 10a, 10b with different pulse lengths Ta and Tb. In particular, the pulse length Tb is shorter than the pulse length Ta in this representation. Nonetheless, both main pulses start at the value x1 and end at the value x2, by corresponding linear mapping of the time t to the computational substitute x.

In the illustration of Figure 3, the parameters k1 to k6 are chosen to be the same for the main pulse 10a and the main pulse 10b. This results in the present example in a temporal compression of the main pulse 10b in comparison to the main pulse 10a, which also results in a changed frequency behavior, in particular in increased spectral components.

The representation in Figure 3 corresponds, for example, to the respective first main pulse of the first sequence of main pulses 10a and second sequence of main pulses 10b. Thus, for example, the starting times of the two main pulses 10a, 10b coincide.

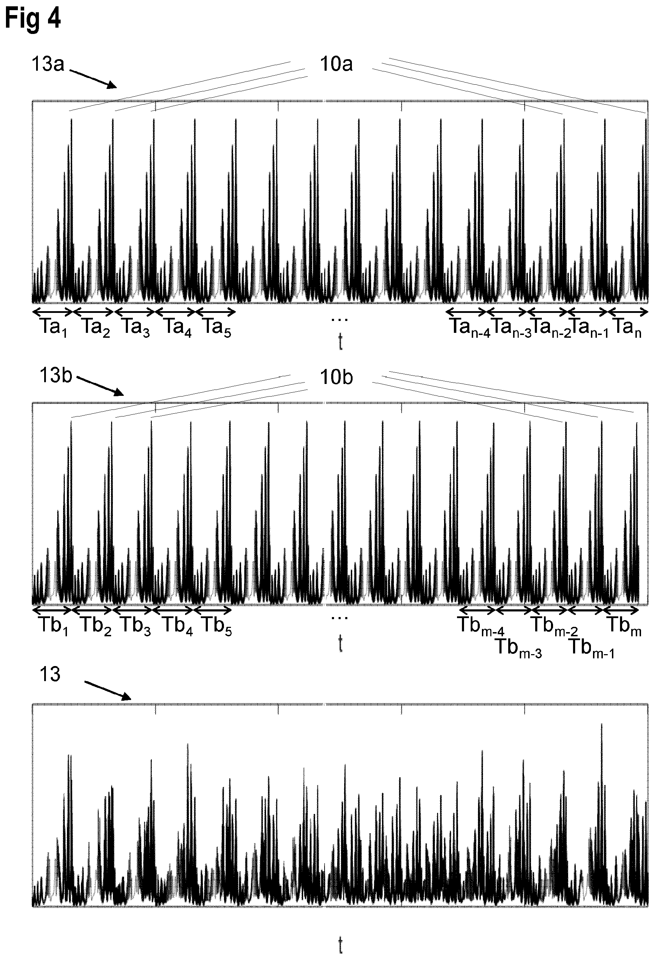

In Figure 4, the upper graph shows a first sequence 13a of main pulses 10a, each comprising a pulse length $Ta_i$ with i = 1 ... n. Similarly, a second sequence 13b of main pulses 10b is shown in the middle graph, each comprising pulse lengths $Tb_i$ with i = 1 ... m.

In the example shown, the pulse lengths $Ta_i$ of the first sequence 13a are constant, while the pulse lengths $Tb_i$ of the second sequence 13b of main pulses 10b decrease linearly.

However, in addition to a linear change, a logarithmic or exponential progression could also be selected. Likewise, it is possible that the pulse length $Ta_i$ also changes accordingly and, in particular, is non-continuous, wherein, according to the improved concept, the pulse lengths of corresponding main pulses in the first and second sequences 13a, 13b are nevertheless different.

The lower graph of Figure 4 shows the signal sequence 13 resulting from a superposition of the first and second sequences 13a, 13b. This signal sequence 13 is, for example, the signal used by the pulse generator 1 to drive the field generation device 2, as described in connection with Figure 1.

For reasons of clarity, a number of 15 and 16 main pulses, respectively, has been selected for the number of main pulses of the first and second sequences 13a, 13b, for example, wherein this number may be considerably higher in practical applications in order to be able to realize corresponding treatment durations. However, this does not exclude the generality of the explanations, but merely serves to make visible the principle of the constantly changing temporal shift and shortening of the main pulses 10b of the second sequence 13b in comparison with the first sequence 13a. In the signal sequence 13, it can be clearly seen that the superposition results in different spectral intensities depending on time, even if a time representation is selected here.

If the individual main pulses within the first or second sequence immediately follow one another, the pulse repetition rate results implicitly from the respective pulse length, wherein the pulse repetition rate thereby changes indirectly proportional to the pulse length. For example:

```
Pulse repetition rate = 1/ pulse length
```

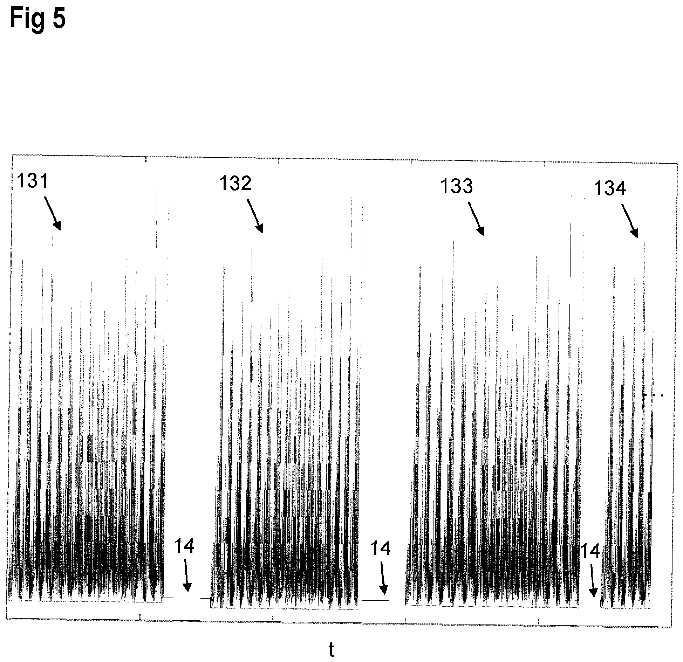

Figure 5 shows the principle time-dependent course of a sequence of superimposed signal sequences of the type described above. For example, the pulse generator 1 is configured to output several signal sequences 131, 132, 133, 134 and so on in succession, wherein these may differ, for example, in the pulse length of the main pulses of the first sequence or the second sequence of main pulses. It is also possible to vary the parameters k1 to k6 between the individual signal sequences 131, 132, 133, 134 and so on, and this permits individual design of the magnetic field to be generated with regard to an optimum effect on the biological tissue applied. Individual defined pause times 14 can be inserted between the signal sequences, which can be designed to be constant or also variable between the different sequences. In principle, the pause time can also be selected to be zero.

The superposition of sequences of main pulses with varying pulse lengths and pulse repetition rates presented in the present disclosure is also applicable to any other forms of electromagnetically pulsed exposures, in particular also with such main pulses which do not directly fall under the above-mentioned formula y(x).

**Claims**

1. A device for influencing biological processes in a living tissue, in particular a human body, for applying a pulsating magnetic field to at least a part of the tissue, with a field generating device (2) for generating the pulsating magnetic field and a pulse generator (1) for driving the field generating device (2) with a signal sequence (13), the pulse generator (1) being adapted to output the signal sequence (13), which is formed from a superposition of a first sequence (13a) of main pulses (10a) and at least one second sequence (13b) of main pulses (10b), the pulse repetition rate of which is between 0.1 and 1000 Hz, in particular is between 3 and 300 Hz, wherein

   - each main pulse (10a, 10b) comprises a pulse length;
   - the pulse length and the pulse repetition rate of the main pulses (10b) of the second sequence (13b) of main pulses (10b) are different from the pulse length and the pulse repetition rate of the main pulses (10a) of the first sequence (13a) of main pulses (10a);
   - the pulse length of the main pulses (10b) of the second sequence (13b) of main pulses (10b) changes monotonically in a time-dependent course, in particular decreases monotonically or increases monotonically;
   - the pulse repetition rate of the main pulses (10b) of the second sequence (13b) of main pulses (10b) changes monotonically in the time-dependent course opposite to the change in the pulse length, in particular increases monotonically or decreases monotonically;
   - an amplitude waveform of a main pulse comprises the following function y(x):

   $$y(x) = k1 + k2 \cdot e^{\sin(x^{k3}) + \sin((x \cdot k4)^{k5}) + x \cdot k6};$$

   - therein mean:

      - x = computational substitute for time t during a main pulse;
      - k1 = offset value;
      - k2 = amplitude factor, k2≠0;
      - k3 = exponent of x, k3≠0;
      - k4 = multiplication factor of x, k4≠0;
      - k5 = exponent of (x*k4), k5≠0;
      - k6 = multiplication factor of x;
      - wherein k1 - k6 are parameters which are freely selectable within certain limits to give different shapes to the amplitude waveform, each main pulse being modulated with sub-pulses (11) by respective selection of the parameters.

2. The device according to claim 1, wherein the computational substitute x depends linearly on the time t.

3. The device according to claim 1 or 2, wherein the computational substitute x for the time t is defined as

   $$x = x1 + \frac{x2 - x1}{T_i} \cdot (t - t0_i),$$

   with

x1 = initial value for x;
x2 = final value for x;
Ti = pulse length of a main pulse; and
t0i = start time of the main pulse.

4. The device according to claim 3, wherein the pulse length Ti of the main pulses (10b) of the second sequence (13b) of main pulses (10b) varies linearly in the time-dependent course.

5. The device according to claim 3, wherein the pulse length Ti of the main pulses (10b) of the second sequence (13b) of main pulses (10b) changes logarithmically in the time-dependent course.

6. The device according to claim 3, wherein the pulse length Ti of the main pulses (10b) of the second sequence of main pulses (10b) changes exponentially in the time-dependent course.

7. The device according to one of the preceding claims, wherein

- the main pulses (10a) of the first sequence (13a) of main pulses (10a) follow one another directly in the time-dependent course, in particular without a pause; and
- the main pulses (10b) of the second sequence (13b) of main pulses (10b) follow one another directly in the time-dependent course, in particular without a pause.

8. The device according to one of the preceding claims, wherein

- the pulse length of the main pulses (10a) of the first sequence of main pulses (10a) changes monotonically in the time-dependent course, in particular decreases monotonically or increases monotonically; and
- the pulse repetition rate of the main pulses (10a) of the first sequence of main pulses (10a) changes monotonically in the time-dependent course opposite to the change in the pulse length of these main pulses (10a), in particular increases monotonically or decreases monotonically.

9. The device according to one of the preceding claims, wherein the pulse generator (1) is adapted to output the signal sequence as a first signal sequence (131) and at least one further signal sequence (132, 133, 134) in temporal succession, the at least one further signal sequence (132, 133, 134) differing from the first signal sequence in at least one of the following:

- the pulse length of the main pulses (10a) of the first sequence of main pulses (10a);
- the pulse length of the main pulses (10b) of the second sequence of main pulses (10b);
- one or more of the parameters k1 - k6.

10. The device according to the preceding claim, wherein the pulse generator (1) is designed to output the at least one further signal sequence after a defined pause time (14) after the output of the first signal sequence.

11. The device according to one of the preceding claims, wherein the parameters of the function y(x) with the properties k3 to k5 are integer and k1, k2 and k6 are selected in decimal increments from the following value ranges:

- 6 < k1, k2, k6 < 6 (particularly in steps of 0.1);
- 6 < k3, k5 < 6 (integer);
- 10< k4 < 10.

**Patentansprüche**

1. Vorrichtung zur Beeinflussung biologischer Abläufe in einem lebenden Gewebe, insbesondere einem menschlichen Körper, zur Beaufschlagung zumindest eines Teils des Gewebes mit einem pulsierenden magnetischem Feld, mit einer Felderzeugungsvorrichtung (2) zur Erzeugung des pulsierenden magnetischen Feldes und einem Impulsgenerator (1) zur Ansteuerung der Felderzeugungsvorrichtung (2) mit einer Signalfolge (13), wobei der Impulsgenerator (1) zur Abgabe der Signalfolge (13) ausgebildet ist, welche gebildet ist aus einer Überlagerung einer ersten Folge (13a) von Hauptimpulsen (10a) und wenigstens einer zweiten Folge (13b) von Hauptimpulsen (10b), deren Impulswiederholungsrate zwischen 0,1 und 1000 Hz liegt, insbesondere zwischen 3 und 300 Hz liegt, wobei

- jeder Hauptimpuls (10a, 10b) eine Impulslänge aufweist;
- die Impulslänge und die Impulswiederholungsrate der Hauptimpulse (10b) der zweiten Folge (13b) von Hauptimpulsen (10b) sich unterscheiden von der Impulslänge und der Impulswiederholungsrate der Hauptimpulse (10a) der ersten Folge (13a) von Hauptimpulsen (10a);
- die Impulslänge der Hauptimpulse (10b) der zweiten Folge (13b) von Hauptimpulsen (10b) sich im zeitlichen Verlauf monoton verändert, insbesondere monoton abfällt oder monoton ansteigt;
- die Impulswiederholungsrate der Hauptimpulse (10b) der zweiten Folge (13b) von Hauptimpulsen (10b) sich im zeitlichen Verlauf entgegengesetzt zur Veränderung der Impulslänge monoton verändert, insbesondere monoton ansteigt oder monoton abfällt;
- der Amplitudenverlauf eines Hauptimpulses die folgende Funktion y(x) aufweist:

$$y(x) = k1 + k2 \cdot e^{\sin(x^{k3}) + \sin((x \cdot k4)^{k5}) + x \cdot k6};$$

- darin bedeuten:

  - x = rechnerische Ersatzgröße für die Zeit t während eines Hauptimpulses;
  - k1 = Offsetwert;
  - k2 = Amplitudenfaktor, k2≠0;
  - k3 = Exponent von x, k3≠0;
  - k4 = Multiplikationsfaktor von x, k4≠0;
  - k5 = Exponent von (x*k4), k5≠0;
  - k6 = Multiplikationsfaktor von x;
  - wobei k1 - k6 Parameter sind, die in gewissen Grenzen frei wählbar sind, um dem Amplitudenverlauf unterschiedliche Formen zu geben, wobei jeder Hauptimpuls durch entsprechende Wahl der Parameter mit Unterimpulsen (11) moduliert ist.

2. Vorrichtung nach Anspruch 1, wobei die rechnerische Ersatzgröße x linear von der Zeit t abhängt.

3. Vorrichtung nach Anspruch 1 oder 2, wobei die rechnerische Ersatzgröße x für die Zeit t definiert ist als

$$x = x1 + \frac{x2 - x1}{T_i} \cdot (t - t0_i),$$

mit

  x1 = Anfangswert für x;
  x2 = Endwert für x;
  Ti = Impulslänge eines Hauptimpulses; und
  t0i = Startzeit des Hauptimpulses.

4. Vorrichtung nach Anspruch 3, wobei sich die Impulslänge Ti der Hauptimpulse (10b) der zweiten Folge (13b) von Hauptimpulsen (10b) im zeitlichen Verlauf linear verändert.

5. Vorrichtung nach Anspruch 3, wobei sich die Impulslänge Ti der Hauptimpulse (10b) der zweiten Folge (13b) von Hauptimpulsen (10b) im zeitlichen Verlauf logarithmisch verändert.

6. Vorrichtung nach Anspruch 3, wobei sich die Impulslänge Ti der Hauptimpulse (10b) der zweiten Folge von Hauptimpulsen (10b) im zeitlichen Verlauf exponentiell verändert.

7. Vorrichtung nach einem der vorhergehenden Ansprüche, wobei

  - die Hauptimpulse (10a) der ersten Folge (13a) von Hauptimpulsen (10a) im zeitlichen Verlauf unmittelbar aufeinanderfolgen, insbesondere ohne Pause; und

- die Hauptimpulse (10b) der zweiten Folge (13b) von Hauptimpulsen (10b) im zeitlichen Verlauf unmittelbar aufeinanderfolgen, insbesondere ohne Pause.

**8.** Vorrichtung nach einem der vorhergehenden Ansprüche, wobei

- die Impulslänge der Hauptimpulse (10a) der ersten Folge von Hauptimpulsen (10a) sich im zeitlichen Verlauf monoton verändert, insbesondere monoton abfällt oder monoton ansteigt; und
- die Impulswiederholungsrate der Hauptimpulse (10a) der ersten Folge von Hauptimpulsen (10a) sich im zeitlichen Verlauf entgegengesetzt zur Veränderung der Impulslänge dieser Hauptimpulse (10a) monoton verändert, insbesondere monoton ansteigt oder monoton abfällt.

**9.** Vorrichtung nach einem der vorhergehenden Ansprüche, wobei der Impulsgenerator (1) ausgebildet ist zur Abgabe der Signalfolge als erste Signalfolge (131) und wenigstens einer weiteren Signalfolge (132, 133, 134) in zeitlicher Abfolge, die sich von der ersten Signalfolge wenigstens in einem der folgenden unterscheidet:

- der Impulslänge der Hauptimpulse (10a) der ersten Folge von Hauptimpulsen (10a);
- der Impulslänge der Hauptimpulse(10b) der zweiten Folge von Hauptimpulsen (10b);
- einem oder mehreren der Parameter k1 - k6.

**10.** Vorrichtung nach dem vorhergehenden Anspruch, wobei der Impulsgenerator (1) ausgebildet ist, die wenigstens eine weitere Signalfolge nach einer definierten Pausenzeit (14) nach der Abgabe der ersten Signalfolge abzugeben.

**11.** Vorrichtung nach einem der vorhergehenden Ansprüche, wobei die Parameter der Funktion y(x) mit den Eigenschaften k3 bis k5 ganzzahlig und k1, k2 und k6 in dezimaler Abstufung aus den folgenden Wertebereichen gewählt werden:

- 6 < k1, k2, k6 < 6 (insbesondere in Schritten von 0,1);
- 6 < k3, k5 < 6 (ganzzahlig);
- 10< k4 < 10.

**Revendications**

**1.** Dispositif destiné à influencer des processus biologiques dans un tissu vivant, en particulier dans le corps humain, pour appliquer un champ magnétique pulsé à au moins une partie du tissu, comprenant un dispositif générateur de champ (2) pour générer le champ magnétique pulsé et un générateur d'impulsions (1) pour commander le dispositif générateur de champ (2) avec une séquence de signaux (13), le générateur d'impulsions (1) étant adapté pour délivrer la séquence de signaux (13), qui est formée à partir d'une superposition d'une première séquence (13a) d'impulsions principales (10a) et d'au moins une deuxième séquence (13b) d'impulsions principales (10b), dont la fréquence de répétition des impulsions est comprise entre 0,1 et 1000 Hz, en particulier entre 3 et 300 Hz, dans lequel

- chaque impulsion principale (10a, 10b) comprend une longueur d'impulsion ;
- la longueur d'impulsion et la fréquence de répétition des impulsions principales (10b) de la deuxième séquence (13b) d'impulsions principales (10b) sont différentes de la longueur d'impulsion et de la fréquence de répétition des impulsions principales (10a) de la première séquence (13a) d'impulsions principales (10a) ;
- la longueur d'impulsion des impulsions principales (10b) de la deuxième séquence (13b) d'impulsions principales (10b) varie de manière monotone en fonction du temps, en particulier diminue de manière monotone ou augmente de manière monotone ;
- la fréquence de répétition des impulsions principales (10b) de la deuxième séquence (13b) d'impulsions principales (10b) varie de manière monotone dans le temps de manière opposée à la variation de la durée d'impulsion, en particulier augmente de manière monotone ou diminue de manière monotone ;
- une forme d'onde d'amplitude d'une impulsion principale comprend la fonction y(x) suivante :

$$y(x) = k1 + k2 \cdot e^{\sin(x^{k3}) + \sin((x \cdot k4)^{k5}) + x \cdot k6} \; ;$$

- où :

- x = substitut computationnel du temps t pendant une impulsion principale ;
- k1 = valeur de décalage ;
- k2 = facteur d'amplitude, k2≠0 ;
- k3 = exposant de x, k3#0 ;
- k4 = facteur de multiplication de x, k4#0 ;
- k5 = exposant de (x*k4), k5≠0 ;
- k6 = facteur de multiplication de x ;
- où k1 - k6 sont des paramètres qui peuvent être librement sélectionnés dans certaines limites pour donner différentes formes à la forme d'onde d'amplitude, chaque impulsion principale étant modulée avec des sous-impulsions (11) par sélection respective des paramètres.

2. Dispositif selon la revendication 1, dans lequel le substitut de calcul x dépend linéairement du temps t.

3. Dispositif selon la revendication 1 ou 2, dans lequel le substitut de calcul x pour le temps t est défini comme suit,

$$x = x1 + \frac{x2 - x1}{T_i} \cdot (t - t0_i),$$

avec

x1 = valeur initiale pour x ;
x2 = valeur finale pour x ;
Ti = longueur d'impulsion d'une impulsion principale ; et
t0i = temps de début de l'impulsion principale.

4. Dispositif selon la revendication 3, dans lequel la longueur d'impulsion Ti des impulsions principales (10b) de la deuxième séquence (13b) d'impulsions principales (10b) varie linéairement en fonction du temps.

5. Dispositif selon la revendication 3, dans lequel la longueur d'impulsion Ti des impulsions principales (10b) de la deuxième séquence (13b) d'impulsions principales (10b) varie de manière logarithmique dans le temps.

6. Dispositif selon la revendication 3, dans lequel la durée d'impulsion Ti des impulsions principales (10b) de la deuxième séquence d'impulsions principales (10b) varie de manière exponentielle dans le temps.

7. Dispositif selon l'une des revendications précédentes, dans lequel

- les impulsions principales (10a) de la première séquence (13a) d'impulsions principales (10a) se succèdent directement dans le temps, en particulier sans pause ; et
- les impulsions principales (10b) de la deuxième séquence (13b) d'impulsions principales (10b) se succèdent directement dans le temps, en particulier sans pause.

8. Dispositif selon l'une des revendications précédentes, dans lequel

- la longueur d'impulsion des impulsions principales (10a) de la première séquence d'impulsions principales (10a) varie de manière monotone dans le temps, en particulier diminue de manière monotone ou augmente de manière monotone ; et
- la fréquence de répétition des impulsions principales (10a) de la première séquence d'impulsions principales (10a) varie de manière monotone dans le temps, à l'opposé de la variation de la durée des impulsions principales (10a), en particulier augmente de manière monotone ou diminue de manière monotone.

9. Dispositif selon l'une des revendications précédentes, dans lequel le générateur d'impulsions (1) est adapté pour émettre la séquence de signaux sous la forme d'une première séquence de signaux (131) et d'au moins une autre séquence de signaux (132, 133, 134) dans un ordre chronologique, la au moins une autre séquence de signaux (132, 133, 134) différant de la première séquence de signaux par au moins l'un des éléments suivants :

- la longueur d'impulsion des impulsions principales (10a) de la première séquence d'impulsions principales (10a) ;

- la longueur d'impulsion des impulsions principales (10b) de la deuxième séquence d'impulsions principales (10b) ;
- un ou plusieurs des paramètres k1 à k6.

10. Dispositif selon la revendication précédente, dans lequel le générateur d'impulsions (1) est conçu pour émettre la au moins une autre séquence de signaux après un temps de pause défini (14) suivant l'émission de la première séquence de signaux.

11. Dispositif selon l'une des revendications précédentes, dans lequel les paramètres de la fonction y(x) avec les propriétés k3 à k5 sont des nombres entiers et k1, k2 et k6 sont sélectionnés par incréments décimaux à partir des plages de valeurs suivantes :

- 6 < k1, k2, k6 < 6 (en particulier par pas de 0,1) ;
- 6 < k3, k5 < 6 (entier) ;
- 10 < k4 < 10.

**Fig 1**

**Fig 2**

10

11

12

x2
t0i + T

t

x1
t0i

# Fig 3

# Fig 4

13a ← 10a

Ta₁ Ta₂ Ta₃ Ta₄ Ta₅   ...   Ta_{n-4} Ta_{n-3} Ta_{n-2} Ta_{n-1} Ta_n
t

13b ← 10b

Tb₁ Tb₂ Tb₃ Tb₄ Tb₅   ...   Tb_{m-4} Tb_{m-2} Tb_m
Tb_{m-3} Tb_{m-1}
t

13 ←

t

Fig 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2050481 B1
- EP 0594655 B1
- EP 0729318 B1
- EP 0995463 B1